# EUROPEAN PATENT SPECIFICATION

(11) **EP 3 813 896 B1**
(45) Date of publication and mention of the grant of the patent: **01.10.2025**
(21) Application number: 19803138.7
(22) Date of filing: 14.05.2019
(51) Int. Cl.: A61L 27/24, A61K 38/39, C12N 5/07

(54) **METHODS AND COMPOSITIONS RELATED TO EXTRACELLULAR MATERIAL DERIVED FROM HYPERTONIC CELL SOLUTIONS**
VERFAHREN UND ZUSAMMENSETZUNGEN IM ZUSAMMENHANG MIT EXTRAZELLULÄREM MATERIAL AUS HYPERTONISCHEN ZELLLÖSUNGEN
MÉTHODES ET COMPOSITIONS ASSOCIÉES À UN MATÉRIEL EXTRACELLULAIRE DÉRIVÉ DE SOLUTIONS DE CELLULES HYPERTONIQUES

(30) Priority: 14.05.2018 US 201862671074 P
(43) Date of publication of application: 05.05.2021
(73) Proprietor: ROOSTERBIO, INC., Frederick, MD 21703 (US)
(72) Inventor: NG, Kelvin, S., Bethesda, MD 20817 (US); ROWLEY, Jonathan, A., Walkersville, MD 21793 (US); LOCK, Lye, Theng, Frederick, MD 21701 (US); RAVISHANKAR, Prarthana, Frederick, MD 21703 (US)
(74) Representative: Berggren Oy
(86) International application number: PCT/US2019/032155
(87) International publication number: WO 2019/222170

(56) References cited:
- WO-A1-2012/003463
- WO-A1-2015/123183
- WO-A1-2015/175457
- US-A1- 2008 081 353
- US-A1- 2013 129 807
- US-A1- 2014 107 036
- US-A1- 2015 057 241
- DEL PICCOLO NUALA ET AL: "Production of Plasma Membrane Vesicles with Chloride Salts and Their Utility as a Cell Membrane Mimetic for Biophysical Characterization of Membrane Protein Interactions", vol. 84, no. 20, 16 October 2012 (2012-10-16), US, pages 8650 - 8655, XP055872700, ISSN: 0003-2700, Retrieved from the Internet <URL:https://www.ncbi.nlm.nih.gov/pmc/articles/PMC3501251/pdf/nihms-414190.pdf> DOI: 10.1021/ac301776j
- UYEN THI TRANG THAN ET AL: "Association of Extracellular Membrane Vesicles with Cutaneous Wound Healing", INTERNATIONAL JOURNAL OF MOLECULAR SCIENCES, vol. 18, no. 5, 1 May 2017 (2017-05-01), pages 956, XP055685049, DOI: 10.3390/ijms18050956
- J-M ESCOLA ET AL: "Selective enrichment of tetraspan proteins on the internal vesicles of multivesicular endosomes and on exosomes secreted by human B-lymphocytes", JOURNAL OF BIOLOGICAL CHEMISTRY, AMERICAN SOCIETY FOR BIOCHEMISTRY AND MOLECULAR BIOLOGY, US, vol. 273, no. 32, 7 August 1998 (1998-08-07), pages 20121 - 20127, XP002163459, ISSN: 0021-9258, DOI: 10.1074/JBC.273.32.20121
- OSWALD, ES ET AL.: "Effects of Hypertonic (NaCI) Two-Dimensional and Three-Dimensional Culture Conditions on the Properties of Cartilage Tissue Engineered from an Expanded Mature Bovine Chondrocyte Source", TISSUE ENGINEERING PART C, METHODS, vol. 17, no. 11, 28 July 2011 (2011-07-28), pages 1041 - 1049, XP055655164

## Description

### CROSS-REFERENCE TO RELATED APPLICATIONS

This application claims benefit of U.S. Provisional Application No. 62/671,074, filed May 14, 2018.

### BACKGROUND

Extracellular material (EM), such as extracellular vesicles (EVs), are emerging as a new class of therapeutics, distinct from other therapeutics. One application is to use EMs to carry and deliver exogenous cargo for therapy, imaging, etc. In this application, EMs may be harvested from any cell type, though most commonly from cell lines that are easy to grow or be genetically modified. Another application is to use EMs from therapeutic cells, from which EMs typically inherit therapeutic properties. EMs with natural therapeutic properties are particularly promising in regenerative medicine: they are a non-living alternative to cell-based therapies-a living therapeutic product-and may tremendously simplify clinical administration and improve the safety profile of regenerative medicine products.

A critical bottleneck in manufacturing EMs is low cellular yield. Extrapolating from animal studies, one human dose of EMs would require billions of cells to generate. What is needed in the art are methods and compositions to boost EM yield from living cells in culture. Such methods and compositions are disclosed herein.

### SUMMARY

The invention is defined in the claims.

Disclosed herein are compositions and methods relating to cells using hypertonic solution to increase extracellular material production.

Specifically, disclosed herein is a pharmaceutical composition comprising an enriched population of extracellular material immersed in a solution comprising an osmolality between 320 and 1000 mOsm/kg.

Also disclosed herein is a kit. The kit can comprise, for example, cells; cell culture media; and a solution bearing an osmolality between 320 mOsm/kg and 20 Osm/kg.

Further disclosed herein is a method for increasing accumulation or production of extracellular material, comprising: immersing cells in a solution bearing an osmolality between 320 and 1000 mOsm/kg; incubating cells in said solution bearing an osmolality between 320 and 1000 mOsm/kg; and collecting and purifying extracellular material released by said cells.

Disclosed herein is a method of treating a subject in need, the method comprising administering to the subject the extracellular material released by the cells, as disclosed herein.

The details of one or more embodiments of the invention are set forth in the accompanying drawings and the description below. Other features, objects, and advantages of the invention will be apparent from the description and drawings, and from the claims.

### DESCRIPTION OF DRAWINGS

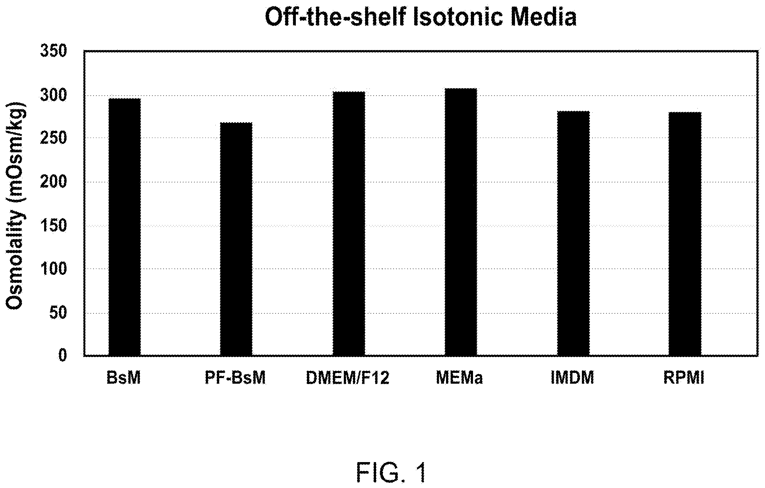

Figure 1 shows an evaluation of existing off-the-shelf basal media show a range of isotonic concentration from 260-310 mOsm/kg.

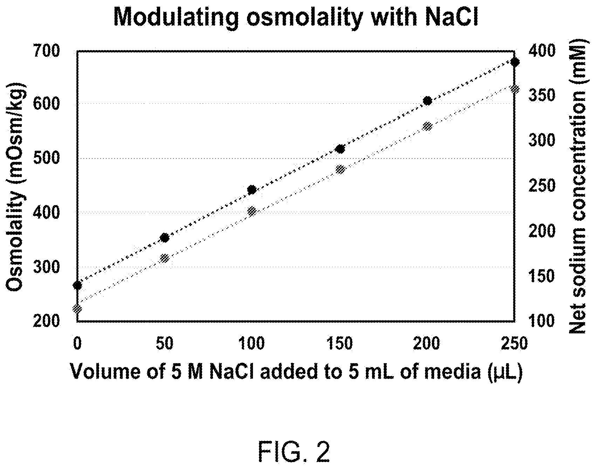

Figure 2 shows an example of NaCl used as solute for modulating media osmolality.

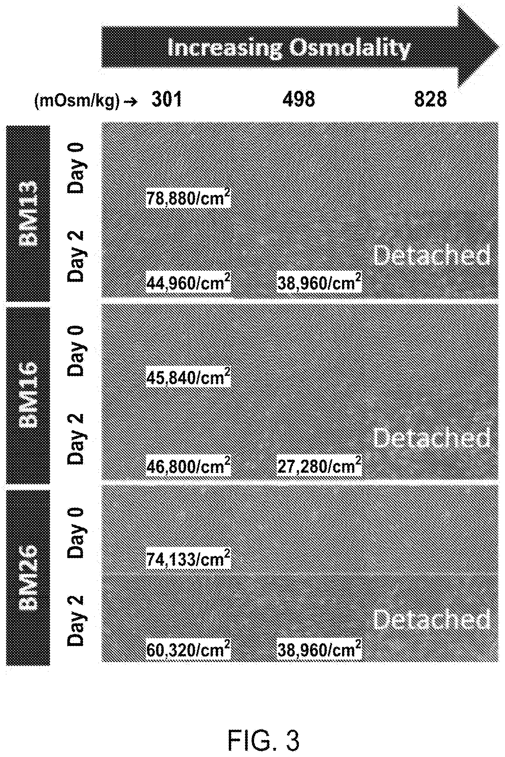

Figure 3 shows hypertonic media can impact long term (2 days) cells survival, hence tuning of osmolality is critical to balance cell viability.

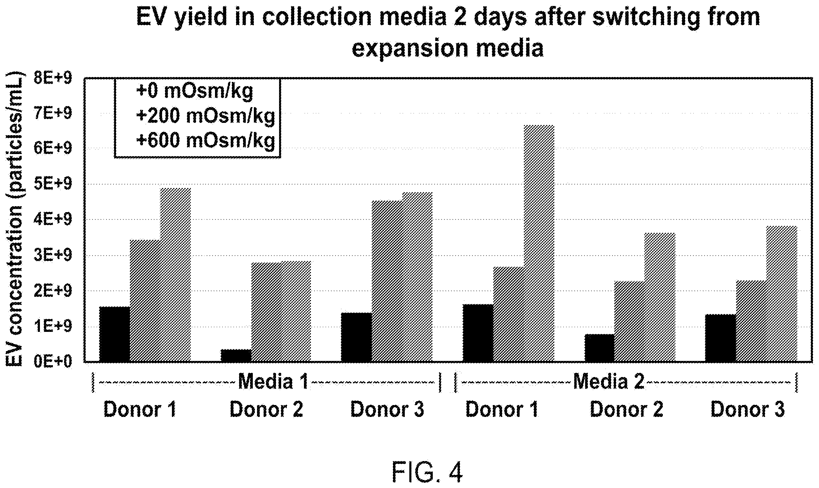

Figure 4 shows extracellular vesicle yield in collection media 2 days after switching from expansion media. 3 different BM-MSC donors and 2 media formulations were evaluated for EV yield. Increasing osmolality (+0, +200 and +600) of collection media (i.e. hypertonic) increases EV yield.

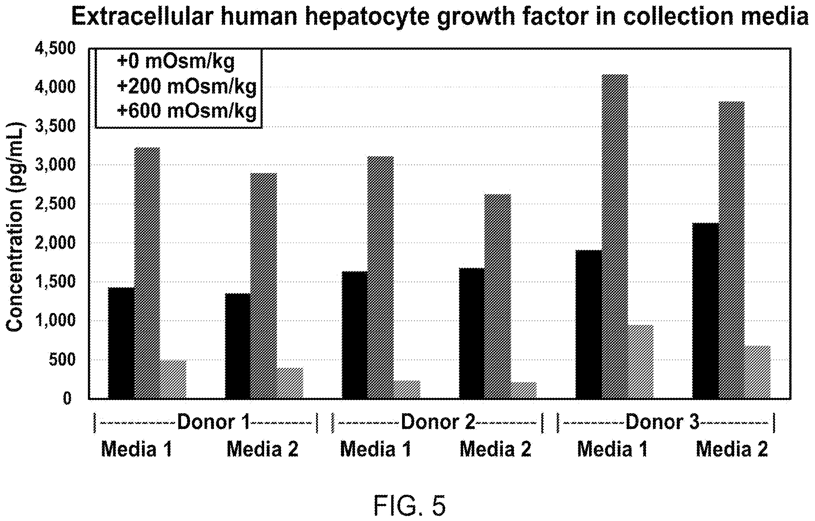

Figure 5 shows extracellular human hepatocyte growth factor in collection media. 3 different BM-MSC donors and 2 media formulations were evaluated for EV yield. Tuning of collection media osmolality (+0, +200 and +600 can control the secretion level of extracellular HGF.
Figure 6 shows extracellular vesicle output as a function of media osmolality. Compared to 48-hour collection using isotonic media (RoosterBasal), hypertonic media can achieve the same yield within 30 minutes, or increase yield by >5X over the same duration.

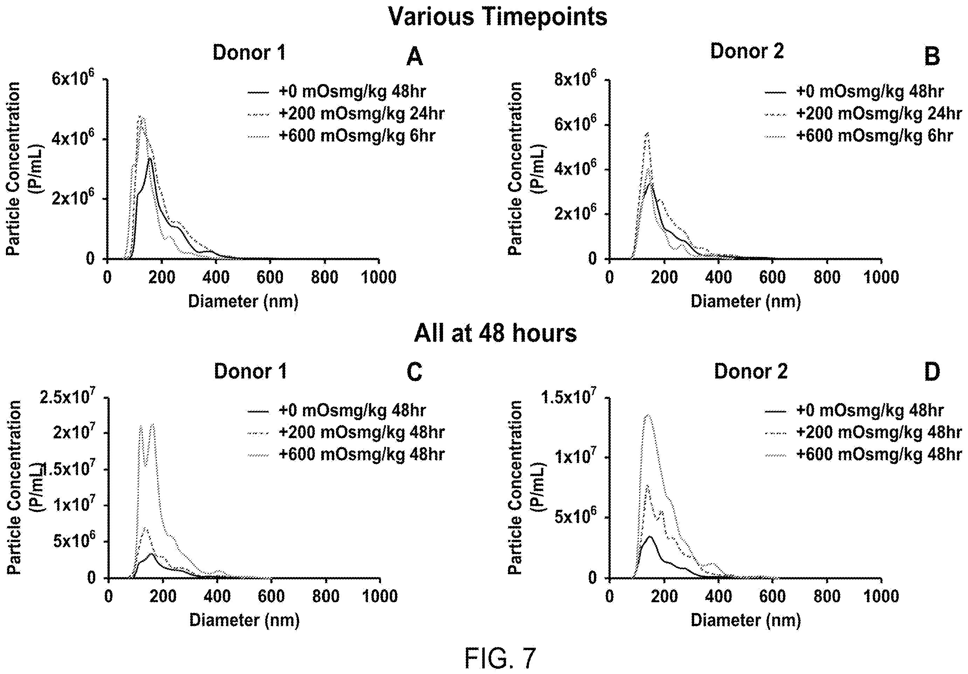

Figure 7A-D shows size distribution of particles in media. All histograms indicate the conditioned media with varying osmolality contains particles in the size range expected for extracellular vesicles. Figure 7A shows donor 1 at all time points. 7B shows donor 2 at all time points. 7C shows donor 1 at 48 hours. 7D shows donor 2 at 48 hours.
Figure 8 shows particles were collected from two harvests alternating between +600mOsm/kg (during harvests) and +0mOsm/kg solution. Similar particle level indicates the feasibility of multiple harvests alternating between medias and using the same cell culture for multiple harvests increasing the cell productivity.
Figure 9 shows particles isolated from conditioned media with different osmolalities contained protein, an important product from conditioned media.

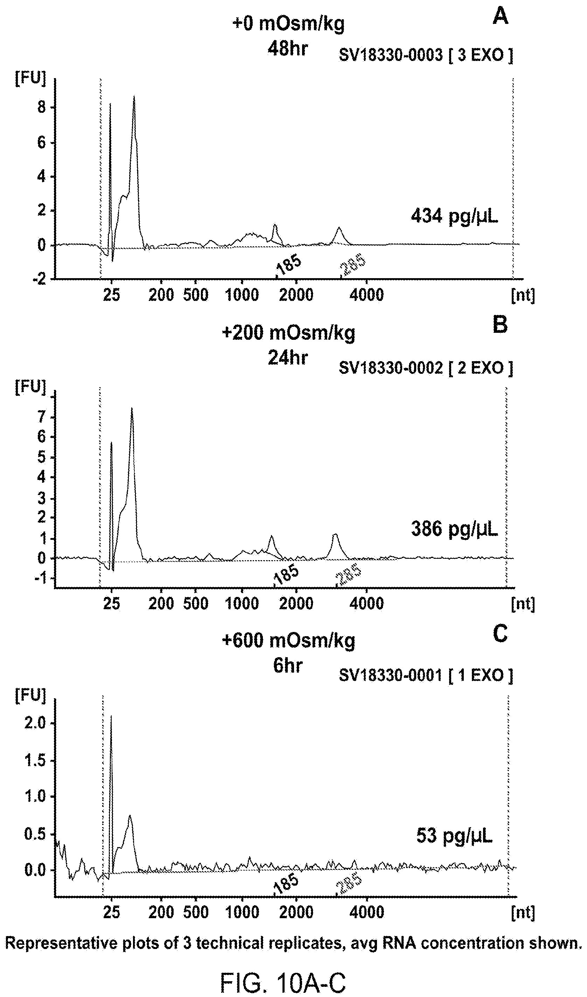

Figure 10A-C shows RNA analysis of collected particles after extracellular vesicle isolation. Different media osmolality (0, +200, +600 mOsm/kg, A-C respectively) resulted in distinct RNA profiles of particles isolated from conditioned media, run on Bioanalyzer. Extracellular vesicles, one product from conditioned media, are known to contain RNA. When the osmolality is increased +200 mOsm/kg, additional particles are generated and the RNA profile is maintained, demonstrating productivity gains in extracellular vesicles. Increasing to +600mOsm/kg, an even greater particle number is generated but the RNA profile is distinct and less RNA is present. Distinct EV populations may be leveraged for different therapeutic applications. Furthermore, these particles contain less RNA cargo and can be utilized as drug delivery vehicles where a payload is loaded.
Figure 11A-B shows conditioned media with normal, +200 mOsm/kg, and +600 mOsm/kg osmolality have higher percent closure compared to control in an *in vitro* wound healing model for donor 1 (11A) and donor 2 (11B). This demonstrates that conditioned media has bioactivity and utility in wound healing and other applications.

### DETAILED DESCRIPTION

### Definitions

Throughout the specification and claims, the following terms take the meanings explicitly associated herein, unless the context clearly dictates otherwise.

The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, without departing from the scope or spirit of the invention.

As used herein, the term "or" is an inclusive "or" operator and is equivalent to the term "and/or" unless the context clearly dictates otherwise.

The term "a," "an," and "the" include plural references. Thus, "a" or "an" or "the" can mean one or more than one. For example, "a" cell and/or extracellular vesicle can mean one cell and/or extracellular vesicle or a plurality of cells and/or extracellular vesicles.

As used herein, "stem cell" refers to a multipotent cell with the potential to differentiate into a variety of other cell types (which perform one or more specific functions), and have the ability to self-renew.

As used herein, "adult stem cells" refer to stem cells that are not embryonic stem cells, and includes neonatal stem cells. By way of example, the adult stem cells include mesenchymal stem cells, also referred to as mesenchymal stromal cells or MSC's.

As used herein, the terms "administering", "introducing", "delivering", "placement" and "transplanting" are used interchangeably and refer to the placement of the extracellular vesicles of the technology into a subject by a method or route that results in at least partial localization of the cells and/or extracellular vesicles at a desired site. The cells and/or extracellular vesicles can be administered by any appropriate route that results in delivery to a desired location in the subject where at least a portion of the cells and/or extracellular vesicles retain their therapeutic capabilities. By way of example, a method of administration includes intravenous administration (i.v.).

As used herein, the term "treating" includes reducing or alleviating at least one adverse effect or symptom of a disease or disorder through introducing in any way a therapeutic composition of the present technology into or onto the body of a subject.

As used herein, "therapeutically effective dose" refers to an amount of a therapeutic agent (e.g., sufficient to bring about a beneficial or desired clinical effect). A dose could be administered in one or multiple administrations (e.g., 2, 3, 4, etc.). However, the precise determination of what would be considered an effective dose may be based on factors individual to each patient, including, but not limited to, the patient's age, size, type or extent of disease, stage of the disease, route of administration, the type or extent of supplemental therapy used, ongoing disease process, and type of treatment desired (e.g., cells and/or extracellular vesicles as a pharmaceutically acceptable preparation) for aggressive vs. conventional treatment.

As used herein, the term "effective amount" refers to the amount of a composition sufficient to effect beneficial or desired results. An effective amount can be administered in one or more administrations, applications or dosages and is not intended to be limited to a particular formulation or administration route.

As used herein, the term "pharmaceutical composition" refers to the combination of an active agent the extracellular vesicles, with, as desired, a carrier, inert or active, making the composition especially suitable for diagnostic or therapeutic use in vitro, in vivo, or ex vivo. As used herein, the terms "pharmaceutically acceptable" or "pharmacologically acceptable" refer to compositions that do not substantially produce adverse reactions, e.g., toxic, allergic, or immunological reactions, when administered to a subject. For example, normal saline is a pharmaceutically acceptable carrier solution.

As used herein, the terms "host", "patient", or "subject" refer to organisms to be treated by the preparations and/or methods of the present technology or to be subject to various tests provided by the technology.

The term "subject" includes animals, preferably mammals, including humans. In some embodiments, the subject is a primate. In other preferred embodiments, the subject is a human. The following examples are provided to demonstrate and further illustrate certain preferred embodiments and aspects of the present technology, and they are not to be construed as limiting the scope of the technology.

The term "cell" as used herein also refers to individual cells, cell lines, primary culture, or cultures derived from such cells unless specifically indicated. A "culture" refers to a composition comprising isolated cells of the same or a different type. Living cells can be found in cell culture.

The term "extracellular material" (also referred to as "EM") refers to cell-derived material, including extracellular vesicles (EVs), that are present in eukaryotic fluids, including blood, lymph, urine, saliva and conditioned medium of cell cultures. This extracellular material can be derived from cellular endosomal and plasma membranes, and can comprise secretory components. Examples of these components comprise, but are not limited to, nucleic acids such as DNAs, RNAs (including miRNA), proteins, polypeptides, carbohydrates, lipids, and small molecules. Extracellular material can be cell-derived small vesicles, and can include exosomes, microvesicles, ectosomes, shedding vesicles, membrane vesicles, plasma membrane vesicles or other membrane-bound assemblies, and apoptotic bodies, viruses, and cells. In certain embodiments, the extracellular material can comprise, consist, or consist essentially of extracellular vesicles.

### General Description

The state-of-the-art practice for producing extracellular material from cultured cells prior to this invention was to culture cells to confluency, rinse to remove existing culture media, and further incubate for 24-72 hours in isotonic media prior to EM collection.

The invention is defined in the claims.

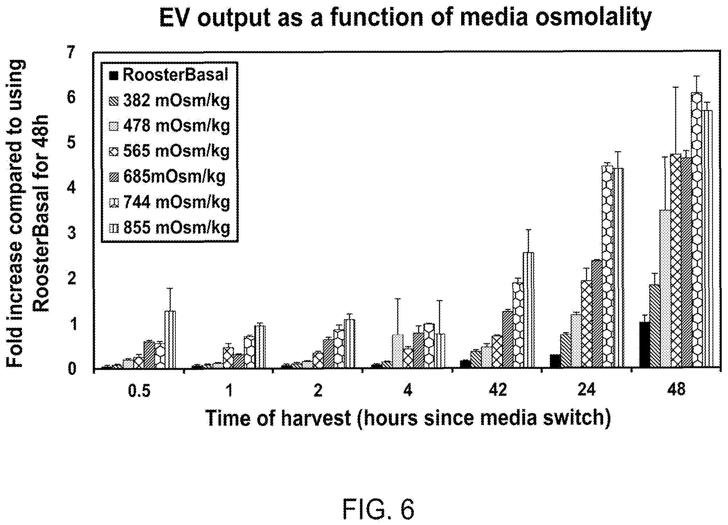

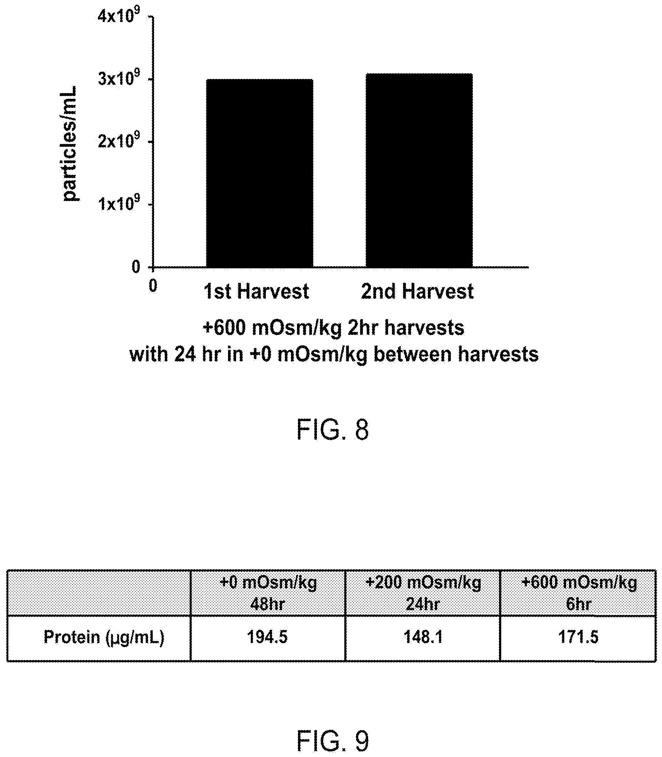

Disclosed herein are methods and compositions that utilize hypertonic solution (greater than 320 mOsm/kg) with living cells to increase the production of EM. Examples of such hypertonic solutions include those spiked with additional sodium chloride. Hypertonic solutions can acutely boost the rate of EM output in a dose-dependent fashion. With the compositions and methods disclosed herein, EM production can be boosted at any point in time, and target yields can be harvested in less time, compared to using isotonic media (Figure 6). This phenomenon was observed across cells from multiple sources/donors and across different media formulations. Multiple harvests can also be carried out, wherein the solution is alternated between different osmolalities (Figure 8). Disclosed herein are methods, kits, devices, and pharmaceutical compositions utilizing the surprising discovery that cells in a hypertonic solution secrete/excrete higher amounts of extracellular material.

Specifically, disclosed herein is a pharmaceutical composition comprising an enriched population of extracellular material immersed in a solution comprising an osmolality between 320 and 1000 mOsm/kg. This is referred to herein as a "hypertonic" solution. The osmolality can be, for example, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 mOsm/kg, or more. As used herein, "isotonic" solution means that the solution has an osmolality between 250 and 320 mOsm/kg. "Hypotonic" means solutions lower than 250 mOsm/kg.

The solution can be formulated from a concentrate. When this is the case, the osmolality of the concentrate can be 1, 2, 3, 4, 5, 6, 7, 8, 9, 10, 11, 12, 13, 14, 15, 16, 17, 18, 19, or 20 or more Osm/kg.

Further disclosed is an enriched population of extracellular material or a fraction thereof suspended in a liquid or colloidal system, frozen, dried, lyophilized, immobilized on the surface of another material, or encapsulated in another material. Also disclosed is an isolated or purified fraction of the pharmaceutical composition.

The extracellular material can be derived from living, or viable, cells. These cells can be found in culture, for example. Examples of cells include, but are not limited to, stem cells, primary cells, immune cells, their daughter cells, or their differentiated derivatives. The cells can be, for example, immortalized primary cells. Cells for use with the invention are described in more detail below.

Also disclosed herein is a kit. The kit can comprise, for example, cells; cell culture media; and a solution bearing an osmolality between 320 mOsm/kg and 20 Osm/kg. Examples of cell culture media that can be used are Dulbecco's Modified Eagle's Medium (DMEM), DMEM/F12, Minimum Essential Medium Eagle Alpha (MEMα), (Roswell Park Memorial Institute) RPMI and Iscove's Modified Dulbecco's Medium (IMDM). Cell culture media contain essential nutrients and minerals to maintain viability of the cultured cells. The cell culture media and the solution can be in the same formula, or formulated together.

The cells can be in suspension; on a solid substrate such as flasks, plates, microcarriers, films, fabrics, or fibers; on or inside a soft substrate such as hydrogels; as aggregates; as single cell solution or a combination thereof. In some embodiments, the cells of the kit have been cultured for 3 or more days without a hypertonic solution, such as in an isotonic solution. By way of example, such culture can be 3, 4, 5, 6, 7, 8, 9, 10, 11, 12, 13, 14, 15, or more days before exposure to the hypertonic solution. The hypertonic solution can contain cell culture media such as DMEM, DMEM/F12, MEMα, RPMI, or IMDM, without added proteins.

Conditioned media can be used with the cells disclosed herein, and can provide a variety of specific attributes or characteristics which are desired and which enhance the production or facilitate the collection of extracellular material. Conditioned medium compositions typically include essential amino acids, salts, vitamins, minerals, trace metals, sugars, lipids and nucleosides. Cell culture medium is able to supply the components necessary to meet the nutritional needs required to grow cells in a controlled environment. Nutrient formulations, pH, and other factors can vary in accordance with parameters such as cell type, cell density, and the culture system employed. A "conditioned medium" is prepared by culturing a first population of cells in a medium, and then harvesting the medium. The conditioned medium (along with anything secreted into the medium by the cells) may then be used to support the growth or elicit response of a second population of cells.

Conditioned medium contains many of the original components of the medium, as well as a variety of cellular metabolites and secreted proteins, including, for example, biologically active growth factors, inflammatory mediators and other extracellular proteins. For example, Figure 9 shows particles isolated from conditioned media with different osmolalities contained protein, an important product from conditioned media.

Further disclosed herein is a method for increasing accumulation or production of extracellular material, comprising: immersing cells in a solution bearing an osmolality between 320 and 1000 mOsm/kg; incubating cells in said solution bearing an osmolality between 320 and 1000 mOsm/kg; and collecting and purifying extracellular material released by said cells.

The increased accumulation or production of extracellular material is higher in said solution bearing an osmolality between 320 and 1000 mOsm/kg than in a solution bearing an osmolality between 250 and 320 mOsm/kg when incubation times are the same. By "higher" is meant that the increase in production from a hypertonic solution as compared to an isotonic solution is 10, 20, 30, 40, 50, 60, 70, 80, 90, or 100% or greater. The increase in production can also be measured as a function of time, with an increase in extracellular material derived from cells in a hypertonic solution being 10, 20, 30, 40, 50, 60, 70, 80, 90, or 100% or greater within a 1, 2, 4, 8, 12, 24, 28, or 72 hour time period when compared to production of EM from cells in an isotonic solution. In one example, the extracellular material can be customized to produce a specific, desired result.

The cells, prior to or after being exposed to the hypertonic solution, can be immersed in a solution bearing an osmolality between and 320 mOsm/kg (an isotonic solution). In one example, the cells can be alternately exposed to a hypertonic and isotonic solution. This alternating between hypertonic and isotonic solutions can occur 2, 3, 4, 5, or more times. The solution can be introduced by fluid flow driven by a pump, gravity, capillary action, or convection. The results of alternating solutions can be seen in Figure 8, for example.

The methods disclosed herein can take place in a hypoxic environment. By "hypoxic environment" is meant where atmospheric oxygen measures between 0% and 20%, or where dissolved oxygen measures between 0% and 80%. The cells and solution can be incubated at a temperature between 4 and 45°C. The pH of said solution, with or without said cells, can measure between 6.5 and 8.0. The cell viability can be 50, 60, 70, 80, 90, or 100%, or any amount in between.

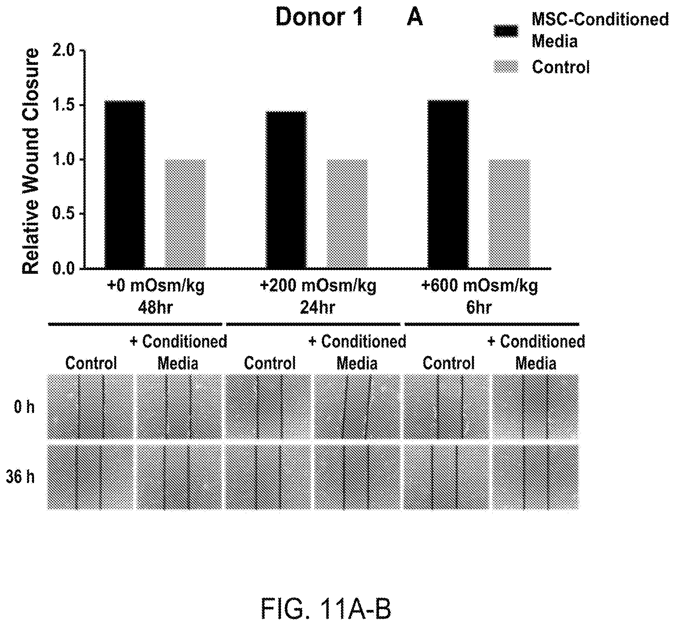

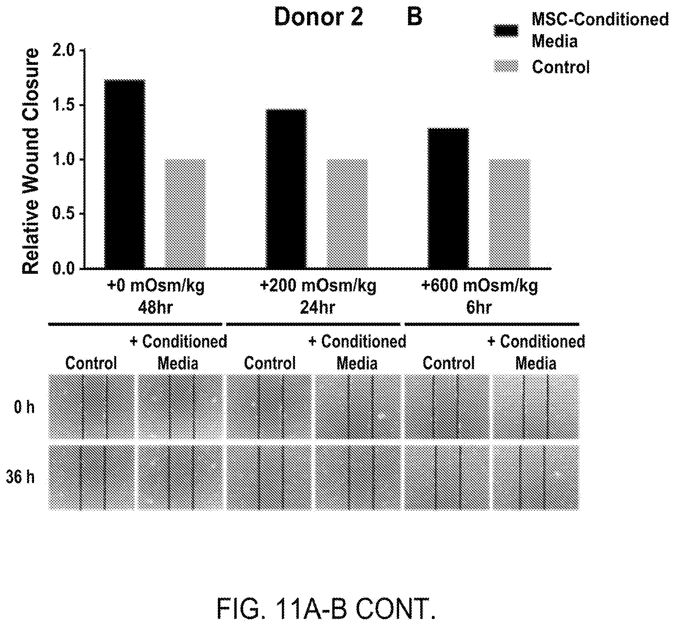

Also disclosed herein is a method of treating a subject in need, the method comprising administering to the subject the extracellular material released by the cells, as disclosed herein. The methods described herein can be used to treat a variety of diseases and disorders, including, but not limited to graft-versus-host disease, pulmonary hypertension, arthritis, ocular trauma, and kidney failure The compositions disclosed herein can be used to promote angiogenesis, wound healing and skin regeneration. For example, the compositions disclosed herein can be disposed on a patch, such as a hydrogel patch. The patch can comprise live cells that produce extracellular vesicles, and the live cells can be disposed in a hypertonic solution. The patch can be used on a subject in need thereof. An example of using the compositions to aid in wound healing can be seen in Figure 11.

As described in the "definitions" section, the extracellular material can be any material extruded from a cell, either by secretion or excretion. This extracellular material can be used for a variety of purposes, including therapeutic and diagnostic purposes. Examples of desirable extracellular material contemplated by the present invention includes, but is not limited to, CD9, CD63, CD81, major histocompatibility complexes or human leukocyte antigens, integrins, tetraspanins, selectins, matrix metalloproteinases, heat shock proteins, histones, amyloid proteins, viral components, cholesterol, phosphatidylserine, nucleic acids (including RNA, DNA, and microRNA), or a combination thereof. The extracellular material can be used as a biomarker or target, and therefore, any material that is suitable for detection can be used. By way of example, the extracellular material can be detected using an assay or diagnostic test. Such assays and diagnostic tests are contemplated herein.

The pharmaceutical composition can also comprise other extracellular material, a cell, a membrane-bound assembly, or a lipid-containing assembly, that is fused to said extracellular material or a fraction thereof. The extracellular material can bear an average particle diameter or an average hydrodynamic diameter between 50 and 1000 nm, or any amount above, below, or in-between. Examples include 50, 100, 200, 300, 400, 500, 600, 700, 800, 900, or 1000 nm. **In** one example, the extracellular material can bear a negative surface charge, or a negative zeta potential.

The pharmaceutical composition can include other components needed to sustain cell viability *in vitro.* Examples of such components include, but are not limited to, lithium, sodium, potassium, calcium, magnesium, chloride, phosphate, acetate, bicarbonate, and/or citrate salts. The pharmaceutical composition can also comprise chemicals that promote angiogenesis or neovascularization, such as angiopoietin (Ang-1), vascular endothelial growth factor (VEGF), platelet-derived growth factor (PDGF), and tissue inhibitors of metalloproteinases (TIMPs). The pharmaceutical composition can also comprise small molecules, contrast agents, preservatives, stabilizers, proteins, lipids, nucleic acids, carbohydrates, their derivatives, or a combination thereof, to affect a permanent or temporary change in physicochemical features, biodistribution, pharmacokinetics, pharmacodynamics, or biological functions of said enriched population of extracellular material or a fraction thereof.

The EM disclosed herein can comprise cargo for delivery to a subject in need thereof. The cargo can be conjugated to EM, embedded within EM, encapsulated within EM, or otherwise carried by EM such as an extracellular vesicle. Alternatively, the EM itself can be the cargo. Thus, as used herein, a reference to a cargo being "present" in EM is understood to include any of the foregoing means of carrying the cargo.

**In** some embodiments, EM, such as an extracellular vesicle, is loaded with 2-5 molecules or copies of a single cargo or two (or more) different cargos. **In** some embodiments, an exosome or pharmaceutical composition thereof is loaded with 1-4,000, 10-4,000, 50-3,500, 100-3,000, 200-2,500, 300-1,500, 500-1,200, 750-1,000, 1-2,000, 1-1,000, 1-500, 10-400, 50-300, 1-250, 1-100, 2-50, 2-25, 2-15, 2-10, 3-50, 3-25, 3-25, 3-10, 4-50, 4-25, 4-15, 4-10, 5-50, 5-25, 5-15, or 5-10 molecules or copies of a single cargo or two (or more) different cargos. The cargo is endogenous or exogenous, and where two or more cargos are present each cargo is independently endogenous or exogenous.

A cargo can be an endogenous cargo, an exogenous cargo, or a combination thereof. Examples of cargos that can be conjugated, embedded, encapsulated within or otherwise carried by an extracellular vesicle described herein include, without limitation, nucleic acid molecules (e.g., DNA, cDNA, antisense oligonucleotides, mRNA, inhibitory RNAs (e.g., anti sense RNAs, miRNAs, small interfering RNAs (siRNAs), short hairpin RNAs (shRNAs), and agomiRs), antagomiRs, primary miRNAs (pri-miRNAs), long non-coding RNAs (IncRNAs), small nuclear RNA (snRNA), small nucleolar RNA (snoRNA), and microbial RNAs), polypeptides (e.g., enzymes, antibodies), lipids, hormones, vitamins, minerals, small molecules, and pharmaceuticals, or any combination thereof.

EM, such as an extracellular vesicle as described herein, can include one or more cargos, wherein the cargo(s) is a therapeutic molecule. Exemplary small molecules include, without limitation, antibiotics, steroids, sterols, peptides, natural products, alkaloids, terpenes, and synthetic molecules.

The cargo can be either diagnostic or therapeutic in nature. When used for diagnostic purposes, the cargo can comprise, for example, an imaging agent. As used herein, an imaging agent is an agent that emits signal directly or indirectly thereby allowing its detection. Imaging agents such as contrast agents and radioactive agents that can be detected using medical imaging techniques such as nuclear medicine scans and magnetic resonance imaging (MRI) are disclosed herein. Also disclosed are imaging agents for fluorescence imaging such as fluorescent dyes or dye-conjugated nanoparticles. In other embodiments, the agent to be delivered is conjugated, or fused to, or mixed or combined with an imaging agent.

A therapeutic molecule can be conjugated to an extracellular vesicle, embedded within an extracellular vesicle, encapsulated within an extracellular vesicle, or otherwise carried by an extracellular vesicle or any combination thereof. Examples of therapeutic agents include, without limitation, mRNAs and/or polypeptides encoded by the mRNAs (e.g., Cre recombinase, insulin, peptide hormones, and enzymes), miRNAs, siRNAs, or miRNA antagonists of therapeutic value, nutrients that may be unstable or have low bioavailability (e.g., vitamins B 1 and B 12, polyunsaturated fatty acids), pharmaceuticals (e.g., antibiotics (such as puromycin, gentamycin, and neomycin), cancer drugs (such as chemotherapeutics, immunotherapies, hormone therapies, and targeted therapies), activators of Toll-like receptors), and molecules to be delivered to macrophages (e.g., to remove or prevent atherosclerotic plaques, or treat macrophage-related cancers), as well as any of the other therapeutic cargo molecules provided herein.

In some embodiments, the therapeutic agent is a biologic. In some embodiments, the biologic is selected from a hormone, allergen, adjuvant, antigen, immunogen, vaccine, interferon, interleukin, growth factor, monoclonal antibody (mAb). In some embodiments, the biologic is a polypeptide, or a peptide.

In some embodiments, the EM can be engineered to partially or completely deplete all or select elements of the cargo, to change the desired payload specifically or in preparation of additional means of cargo loading. Methods of engineering EM, such as extracellular vesicles, are known in the prior art and are described in Dhruvitkumar et al. (2017) *Low active loading of cargo into engineered extracellular vesicles results in inefficient miRNA mimic delivery,* Journal of Extracellular Vesicles, 6:1, herein incorporated by reference in its entirety for its teaching concerning EM and cargo.

The cells for use with the compositions disclosed herein described herein can be any cells capable of culture, and can include living, viable cells. The cells can be modified. For example, the cells can be genetically modified to affect a permanent or temporary change in physicochemical features, biodistribution, pharmacokinetics, pharmacodynamics, or biological functions of said extracellular material.

In some embodiments, the disclosed composition contains stem cells or progenitor cells. Pluripotent stem cells, adult stem cells, blastocyst-derived stem cells, gonadal ridge-derived stem cells, teratoma-derived stem cells, totipotent stem cells, multipotent stem cells, oncostatin-independent stem cell (OISCs), embryonic stem cells (ES), embryonic germ cells (EG), and embryonic carcinoma cells (EC) are all examples of stem cells. Stem cells can have a variety of different properties and categories of these properties. For example in some forms stem cells are capable of proliferating for at least 10, 15, 20, 30, or more passages in an undifferentiated state. In some forms the stem cells can proliferate for more than a year without differentiating. Stem cells can also maintain a normal karyotype while proliferating and/or differentiating. Stem cells can also be capable of retaining the ability to differentiate into mesoderm, endoderm, and ectoderm tissue, including germ cells, eggs and sperm. Some stem cells can also be cells capable of indefinite proliferation in vitro in an undifferentiated state. Some stem cells can also maintain a normal karyotype through prolonged culture. Some stem cells can maintain the potential to differentiate to derivatives of all three embryonic germ layers (endoderm, mesoderm, and ectoderm) even after prolonged culture. Some stem cells can form any cell type in the organism. Some stem cells can form embryoid bodies under certain conditions, such as growth on media which do not maintain undifferentiated growth. Some stem cells can form chimeras through fusion with a blastocyst, for example. Some stem cells can be induced or transformed from non-stem cells by genetic or chemical means.

Some stem cells can be defined by a variety of markers. For example, some stem cells express alkaline phosphatase. Some stem cells express SSEA-1, SSEA-3, SSEA-4, TRA-1-60, and/or TRA-1-81. Some stem cells do not express SSEA-1, SSEA-3, SSEA-4, TRA-1-60, and/or TRA-1-81. Some stem cells express Oct4, Sox2, and Nanog. It is understood that some stem cells will express these at the mRNA level, and still others will also express them at the protein level, on for example, the cell surface or within the cell.

In some embodiments, the disclosed composition comprises a cell other than a stem cell. The adult human body produces many different cell types. These different cell types include, but are not limited to, Keratinizing Epithelial Cells, Wet Stratified Barrier Epithelial Cells, Exocrine Secretory Epithelial Cells, Hormone Secreting Cells, Epithelial Absorptive Cells (Gut, Exocrine Glands and Urogenital Tract), Metabolism and Storage cells, Barrier Function Cells (Lung, Gut, Exocrine Glands and Urogenital Tract), Epithelial Cells Lining Closed Internal Body Cavities, Ciliated Cells with Propulsive Function, Extracellular Matrix Secretion Cells, Contractile Cells, Blood and Immune System Cells, Sensory Transducer Cells, Autonomic Neuron Cells, Sense Organ and Peripheral Neuron Supporting Cells, Central Nervous System Neurons and Glial Cells, Lens Cells, Pigment Cells, Germ Cells, and Nurse Cells.

Cells of the human body include Keratinizing Epithelial Cells, Epidermal keratinocyte (differentiating epidermal cell), Epidermal basal cell (stem cell), Keratinocyte of fingernails and toenails, Nail bed basal cell (stem cell), Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell (stem cell), Wet Stratified Barrier Epithelial Cells, Surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, Urinary epithelium cell (lining bladder and urinary ducts), Exocrine Secretory Epithelial Cells, Salivary gland mucous cell (polysaccharide-rich secretion), Salivary gland serous cell (glycoprotein enzyme-rich secretion), Von Ebner's gland cell in tongue (washes taste buds), Mammary gland cell (milk secretion), Lacrimal gland cell (tear secretion), Ceruminous gland cell in ear (wax secretion), Eccrine sweat gland dark cell (glycoprotein secretion), Eccrine sweat gland clear cell (small molecule secretion), Apocrine sweat gland cell (odoriferous secretion, sex-hormone sensitive), Gland of Moll cell in eyelid (specialized sweat gland), Sebaceous gland cell (lipid-rich sebum secretion), Bowman's gland cell in nose (washes olfactory epithelium), Brunner's gland cell in duodenum (enzymes and alkaline mucus), Seminal vesicle cell (secretes seminal fluid components, including fructose for swimming sperm), Prostate gland cell (secretes seminal fluid components), Bulbourethral gland cell (mucus secretion), Bartholin's gland cell (vaginal lubricant secretion), Gland of Littre cell (mucus secretion), Uterus endometrium cell (carbohydrate secretion), Isolated goblet cell of respiratory and digestive tracts (mucus secretion), Stomach lining mucous cell (mucus secretion), Gastric gland zymogenic cell (pepsinogen secretion), Gastric gland oxyntic cell (HCl secretion), Pancreatic acinar cell (bicarbonate and digestive enzyme secretion), Paneth cell of small intestine (lysozyme secretion), Type II pneumocyte of lung (surfactant secretion), Clara cell of lung, Hormone Secreting Cells, Anterior pituitary cell secreting growth hormone, Anterior pituitary cell secreting follicle-stimulating hormone, Anterior pituitary cell secreting luteinizing hormone, Anterior pituitary cell secreting prolactin, Anterior pituitary cell secreting adrenocorticotropic hormone, Anterior pituitary cell secreting thyroid-stimulating hormone, Intermediate pituitary cell secreting melanocyte-stimulating hormone, Posterior pituitary cell secreting oxytocin, Posterior pituitary cell secreting vasopressin, Gut and respiratory tract cell secreting serotonin, Gut and respiratory tract cell secreting endorphin, Gut and respiratory tract cell secreting somatostatin, Gut and respiratory tract cell secreting gastrin, Gut and respiratory tract cell secreting secretin, Gut and respiratory tract cell secreting cholecystokinin, Gut and respiratory tract cell secreting insulin, Gut and respiratory tract cell secreting glucagon, Gut and respiratory tract cell secreting bombesin, Thyroid gland cell secreting thyroid hormone, Thyroid gland cell secreting calcitonin, Parathyroid gland cell secreting parathyroid hormone, Parathyroid gland oxyphil cell, Adrenal gland cell secreting epinephrine, Adrenal gland cell secreting norepinephrine, Adrenal gland cell secreting steroid hormones (mineralcorticoids and gluco corticoids), Leydig cell of testes secreting testosterone, Theca interna cell of ovarian follicle secreting estrogen, Corpus luteum cell of ruptured ovarian follicle secreting progesterone, Kidney juxtaglomerular apparatus cell (renin secretion), Macula densa cell of kidney, Peripolar cell of kidney, Mesangial cell of kidney, Epithelial Absorptive Cells (Gut, Exocrine Glands and Urogenital Tract), Intestinal brush border cell (with microvilli), Exocrine gland striated duct cell, Gall bladder epithelial cell, Kidney proximal tubule brush border cell, Kidney distal tubule cell, Ductulus efferens nonciliated cell, Epididymal principal cell, Epididymal basal cell, Metabolism and Storage Cells, Hepatocyte (liver cell), White fat cell, Brown fat cell, Liver lipocyte, Barrier Function Cells (Lung, Gut, Exocrine Glands and Urogenital Tract), Type I pneumocyte (lining air space of lung), Pancreatic duct cell (centroacinar cell), Nonstriated duct cell (of sweat gland, salivary gland, mammary gland, etc.), Kidney glomerulus parietal cell, Kidney glomerulus podocyte, Loop of Henle thin segment cell (in kidney), Kidney collecting duct cell, Duct cell (of seminal vesicle, prostate gland, etc.), Epithelial Cells Lining Closed Internal Body Cavities, Blood vessel and lymphatic vascular endothelial fenestrated cell, Blood vessel and lymphatic vascular endothelial continuous cell, Blood vessel and lymphatic vascular endothelial splenic cell, Synovial cell (lining joint cavities, hyaluronic acid secretion), Serosal cell (lining peritoneal, pleural, and pericardial cavities), Squamous cell (lining perilymphatic space of ear), Squamous cell (lining endolymphatic space of ear), Columnar cell of endolymphatic sac with microvilli (lining endolymphatic space of ear), Columnar cell of endolymphatic sac without microvilli (lining endolymphatic space of ear), Dark cell (lining endolymphatic space of ear), Vestibular membrane cell (lining endolymphatic space of ear), Stria vascularis basal cell (lining endolymphatic space of ear), Stria vascularis marginal cell (lining endolymphatic space of ear), Cell of Claudius (lining endolymphatic space of ear), Cell of Boettcher (lining endolymphatic space of ear), Choroid plexus cell (cerebrospinal fluid secretion), Pia-arachnoid squamous cell, Pigmented ciliary epithelium cell of eye, Nonpigmented ciliary epithelium cell of eye, Corneal endothelial cell, Ciliated Cells with Propulsive Function, Respiratory tract ciliated cell, Oviduct ciliated cell (in female), Uterine endometrial ciliated cell (in female), Rete testis cilated cell (in male), Ductulus efferens ciliated cell (in male), Ciliated ependymal cell of central nervous system (lining brain cavities), Extracellular Matrix Secretion Cells, Ameloblast epithelial cell (tooth enamel secretion), Planum semilunatum epithelial cell of vestibular apparatus of ear (proteoglycan secretion), Organ of Corti interdental epithelial cell (secreting tectorial membrane covering hair cells), Loose connective tissue fibroblasts, Corneal fibroblasts, Tendon fibroblasts, Bone marrow reticular tissue fibroblasts, Other (nonepithelial) fibroblasts, Blood capillary pericyte, Nucleus pulposus cell of intervertebral disc, Cementoblast/cementocyte (tooth root bonelike cementum secretion), Odontoblast/odontocyte (tooth dentin secretion), Hyaline cartilage chondrocyte, Fibrocartilage chondrocyte, Elastic cartilage chondrocyte, Osteoblast/osteocyte, Osteoprogenitor cell (stem cell of osteoblasts), Hyalocyte of vitreous body of eye, Stellate cell of perilymphatic space of ear, Contractile Cells, Red skeletal muscle cell (slow), White skeletal muscle cell (fast), Intermediate skeletal muscle cell, Muscle spindle -- nuclear bag cell, Muscle spindle -- nuclear chain cell, Satellite cell (stem cell), Ordinary heart muscle cell, Nodal heart muscle cell, Purkinje fiber cell, Smooth muscle cell (various types), Myoepithelial cell of iris, Myoepithelial cell of exocrine glands, Blood and Immune System Cells, Erythrocyte (red blood cell), Megakaryocyte, Monocyte, Connective tissue macrophage (various types), Epidermal Langerhans cell, Osteoclast (in bone), Dendritic cell (in lymphoid tissues), Microglial cell (in central nervous system), Neutrophil, Eosinophil, Basophil, Mast cell, Helper T lymphocyte cell, Suppressor T lymphocyte cell, Killer T lymphocyte cell, IgM B lymphocyte cell, IgG B lymphocyte cell, IgA B lymphocyte cell, IgE B lymphocyte cell, Killer cell, Stem cells and committed progenitors for the blood and immune system (various types), Sensory Transducer Cells, Photoreceptor rod cell of eye, Photoreceptor blue-sensitive cone cell of eye, Photoreceptor green-sensitive cone cell of eye, Photoreceptor red-sensitive cone cell of eye, Auditory inner hair cell of organ of Corti, Auditory outer hair cell of organ of Corti, Type I hair cell of vestibular apparatus of ear (acceleration and gravity), Type II hair cell of vestibular apparatus of ear (acceleration and gravity), Type I taste bud cell, Olfactory neuron, Basal cell of olfactory epithelium (stem cell for olfactory neurons), Type I carotid body cell (blood pH sensor), Type II carotid body cell (blood pH sensor), Merkel cell of epidermis (touch sensor), Touch-sensitive primary sensory neurons (various types), Cold-sensitive primary sensory neurons, Heat-sensitive primary sensory neurons, Pain-sensitive primary sensory neurons (various types), Proprioceptive primary sensory neurons (various types), Autonomic Neuron Cells, Cholinergic neural cell (various types), Adrenergic neural cell (various types), Peptidergic neural cell (various types), Sense Organ and Peripheral Neuron Supporting Cells, Inner pillar cell of organ of Corti, Outer pillar cell of organ of Corti, Inner phalangeal cell of organ of Corti, Outer phalangeal cell of organ of Corti, Border cell of organ of Corti, Hensen cell of organ of Corti, Vestibular apparatus supporting cell, Type I taste bud supporting cell, Olfactory epithelium supporting cell, Schwann cell, Satellite cell (encapsulating peripheral nerve cell bodies), Enteric glial cell, Central Nervous System Neurons and Glial Cells, Neuron cell (large variety of types, still poorly classified), Astrocyte glial cell (various types), Oligodendrocyte glial cell, Lens Cells, Anterior lens epithelial cell, Crystallin-containing lens fiber cell, Pigment Cells, Melanocyte, Retinal pigmented epithelial cell, Germ Cells, Oogonium/oocyte, Spermatocyte, Spermatogonium cell (stem cell for spermatocyte), Nurse Cells, Ovarian follicle cell, Sertoli cell (in testis), and Thymus epithelial cell.

In some cases, the cells are mesenchymal stem cells (MSCs) or bone marrow stromal cells (BMSCs). These terms are used synonymously throughout herein. MSCs are of interest because they are easily isolated from a small aspirate of bone marrow, or other mesenchymal stem cell sources, and they readily generate single-cell derived colonies. Bone marrow cells may be obtained from iliac crest, femora, tibiae, spine, rib, knee or other mesenchymal tissues. Other sources of MSCs include embryonic yolk sac, placenta, umbilical cord, skin, fat, synovial tissue from joints, and blood. The presence of MSCs in culture colonies may be verified by specific cell surface markers which are identified with monoclonal antibodies. See U.S. Pat. Nos. 5,486,359 and 7,153,500. The single-cell derived colonies can be expanded through as many as 50 population doublings in about 10 weeks, and can differentiate into osteoblasts, adipocytes, chondrocytes (Friedenstein et al., 1970 Cell Tissue Kinet. 3:393-403; Castro-Malaspina, et al., 1980 Blood 56:289-301; Beresford et al., 1992 J. Cell Sci. 102:341-351; Prockop, 1997 Science 276:71-74), myocytes (Wakitani et al, 1995 Muscle Nerve 18:1417-1426), astrocytes, oligodendrocytes, and neurons (Azizi et al., 1998 Proc. Natl. Acad. Sci. USA 95:3908-3913); Kopen et al 1999 Proc. Natl. Acad. Sci. USA 96:10711-10716; Chopp et al., 2000 Neuroreport II 300 1-3005; Woodbury et al., 2000 Neuroscience Res. 61:364-370). In rare instances, the cells can differentiate into cells of all three germlines. Thus, MSCs serve as progenitors for multiple mesenchymal cell lineages including bone, cartilage, ligament, tendon, adipose, muscle, cardiac tissue, stroma, dermis, and other connective tissues. See U.S. Pat. Nos. 6,387,369 and 7,101,704. For these reasons, MSCs currently are being tested for their potential use in cell and gene therapy of a number of human diseases (Horwitz et al., 1999 Nat. Med. 5:309-313; Caplan, et al. 2000 Clin. Orthoped. 379:567-570).

In some cases, MSCs can be defined by a variety of markers. For example, MSCs can be positive for CD73, CD90, CD166 and negative for CD14, CD34 and CD45.

The cells can be derived from a human or other animal. For example, cells can originate from a mouse, guinea pig, rat, cattle, horses, pigs, sheep, or goat. In some embodiments, the cells originate from non-human primates. In some cases, the cells are used as autologous or allogenic treatment.

A number of embodiments of the invention have been described. Nevertheless, it will be understood that various modifications may be made without departing from the scope of the invention. Accordingly, other embodiments are within the scope of the following claims.

### EXAMPLES

### Example 1: Extracellular Material Derived from Mesenchymal Stem Cells in Hypertonic Solution

As referred to herein, "hypertonic solution" is formulated to a total osmolality of >350 mOsm/kg, that is used for collecting or extracting extracellular vesicles (EVs) or other cell-derived materials from cells. The EVs referred herein include exosomes, microvesicles, and any other EVs distinct from apoptotic bodies. The cells referred to herein can be human mesenchymal stem cells (hMSCs).

Application of this invention is not limited by specific demands of individual cell types: formulation of the Hypertonic Solution can be varied to suit various contexts as long as the total osmolality exceeds 350 mOsm/kg. One context is where cell attachment or viability is desired. Cells remained attached/viable when the osmolality of the solution was increased up to 443 mOsm/kg; when the osmolality of the solution was increased up to 658 Osm/kg; and when the osmolality of IMDM (301 mOsm/kg) was increased up to 498 mOsm/kg. In these scenarios, EV yield increased by >10X within 30 minutes, compared to isotonic media. Furthermore, the disclosed Hypertonic Solution yielded more EVs within 12 hours than isotonic media could yield over 48 hours (Figure 6).

Cell viability for different donors and formulations can be monitored and established for specific applications, and one of skill in the art will be able to determine the proper osmolality of a specific solution for a determined use. Furthermore, one example of the Hypertonic Solution disclosed herein yielded more EVs within 30 minutes than isotonic media could yield over 48 hours (Figure 6).

In either context, more EVs are produced faster-a unique feature which was unexpected in these experiments (Figure 6). In particular, EVs were quantified after removing apoptotic bodies by centrifugation of cell culture supernatants at 2,000 rcf for 20 minutes. Hence, while media hypertonicity may decrease cell attachment or viability, the increase in EVs is not due to an increase in apoptotic bodies.

Unless defined otherwise, all technical and scientific terms used herein have the same meanings as commonly understood by one of skill in the art to which the disclosed invention belongs.

Those skilled in the art will recognize, or be able to ascertain using no more than routine experimentation, many equivalents to the specific embodiments of the invention described herein. Such equivalents are intended to be encompassed by the following claims.

## Claims

1. A pharmaceutical composition comprising an enriched population of extracellular material derived from living isolated or cultured cells, wherein said extracellular material is released from said cells by immersing said cells in a solution comprising an osmolality between 380 and 1000 mOsm/kg, such as 480-860 mOsm/kg.

2. The pharmaceutical composition of claim 1, wherein said solution comprises components capable of sustaining cell viability *in vitro,* the components comprising lithium, sodium, potassium, calcium, magnesium, chloride, phosphate, acetate, bicarbonate, and/or citrate salts.

3. The pharmaceutical composition of any one of claims 1-2, wherein the cells comprise adult stem cells, and/or the extracellular material comprises extracellular vesicles.

4. The pharmaceutical composition of any one of claims 1-3, wherein said enriched population of extracellular material comprises therapeutic molecule.

5. The pharmaceutical composition of claim 4, wherein said therapeutic molecule is CD9, CD63, CD81, cholesterol, phosphatidylserine, or a combination thereof.

6. The pharmaceutical composition of claim 4, wherein said therapeutic molecule is angiopoietin (Ang-1).

7. The pharmaceutical composition of claim 4, wherein said therapeutic molecule_comprises nucleic acid molecules, polypeptides, lipids, hormones, vitamins, minerals, small molecules, and pharmaceuticals, or any combination thereof.

8. An isolated or purified fraction of the pharmaceutical composition of any one of claims 1-7.

9. The pharmaceutical composition of any one of claims 1-8, wherein said enriched population of extracellular material or a fraction thereof is suspended in a liquid or colloidal system, frozen, dried, lyophilized, immobilized on the surface of another material, or encapsulated in another material.

10. The pharmaceutical composition of any one of claims 1-9, wherein the pharmaceutical composition comprises small molecules, contrast agents, preservatives, stabilizers, proteins, lipids, nucleic acids, carbohydrates, their derivatives, or a combination thereof, to affect a permanent or temporary change in physicochemical features, biodistribution, pharmacokinetics, pharmacodynamics, or biological functions of said enriched population of extracellular material or a fraction thereof.

11. The pharmaceutical composition of any one of claims 1-10, further comprising other extracellular material, a cell, a membrane-bound assembly, or a lipid-containing assembly, that is fused to said extracellular material or a fraction thereof.

12. The pharmaceutical composition of claims 1-11, wherein said extracellular material bears an average particle diameter or an average hydrodynamic diameter between 50 and 1000 nm.

13. The pharmaceutical composition of any one of claims 1-12, wherein said extracellular material bears a negative surface charge, or a negative zeta potential.

14. The pharmaceutical composition of any one of claims 1-13, wherein the pharmaceutical composition is in patch form.

15. A method of enriching cells for extracellular material, comprising:
a) immersing the cells in a solution bearing an osmolality between 380 and 1000 mOsm/kg;
b) incubating cells in said solution bearing an osmolality between 380 and 1000 mOsm/kg; and
c) collecting and purifying extracellular material released by said cells.

16. The pharmaceutical composition of any one of claims 1-14 or the method of claim 15, wherein the cells comprise mesenchymal stem cells.

## Patentansprüche

1. Pharmazeutische Zusammensetzung, umfassend eine angereicherte Population von extrazellulärem Material, abgeleitet von lebenden isolierten oder kultivierten Zellen, wobei das extrazelluläre Material aus den Zellen durch Eintauchen der Zellen in eine Lösung freigesetzt wird, die eine Osmolalität zwischen 380 und 1000 mOsm/kg, wie 480-860 mOsm/kg, aufweist.

2. Pharmazeutische Zusammensetzung nach Anspruch 1, wobei die Lösung Komponenten umfasst, die in der Lage sind, die Lebensfähigkeit der Zellen *in vitro* aufrechtzuerhalten, wobei die Komponenten Lithium-, Natrium-, Kalium-, Calcium-, Magnesium-, Chlorid-, Phosphat-, Acetat-, Bicarbonat- und/oder Citratsalze umfassen.

3. Pharmazeutische Zusammensetzung nach einem der Ansprüche 1 bis 2, wobei die Zellen adulte Stammzellen umfassen und/oder das extrazelluläre Material extrazelluläre Vesikel umfasst.

4. Pharmazeutische Zusammensetzung nach einem der Ansprüche 1 bis 3, wobei die angereicherte Population von extrazellulärem Material ein therapeutisches Molekül umfasst.

5. Pharmazeutische Zusammensetzung nach Anspruch 4, wobei das therapeutische Molekül CD9, CD63, CD81, Cholesterin, Phosphatidylserin oder eine Kombination davon ist.

6. Pharmazeutische Zusammensetzung nach Anspruch 4, wobei das therapeutische Molekül Angiopoietin (Ang-1) ist.

7. Pharmazeutische Zusammensetzung nach Anspruch 4, wobei das therapeutische Molekül Nukleinsäuremoleküle, Polypeptide, Lipide, Hormone, Vitamine, Mineralien, kleine Moleküle und Pharmazeutika oder irgendeine Kombination davon umfasst.

8. Isolierte oder gereinigte Fraktion der pharmazeutischen Zusammensetzung nach einem der Ansprüche 1 bis 7.

9. Pharmazeutische Zusammensetzung nach einem der Ansprüche 1 bis 8, wobei die angereicherte Population von extrazellulärem Material oder eine Fraktion davon in einem flüssigen oder kolloidalen System suspendiert, gefroren, getrocknet, gefriergetrocknet, auf der Oberfläche eines anderen Materials immobilisiert oder in einem anderen Material verkapselt ist.

10. Pharmazeutische Zusammensetzung nach einem der Ansprüche 1 bis 9, wobei die pharmazeutische Zusammensetzung kleine Moleküle, Kontrastmittel, Konservierungsmittel, Stabilisatoren, Proteine, Lipide, Nukleinsäuren, Kohlenhydrate, deren Derivate oder eine Kombination davon umfasst, um eine dauerhafte oder vorübergehende Veränderung der physikalisch-chemischen Merkmale, der biologischen Verteilung, der Pharmakokinetik, der Pharmakodynamik oder der biologischen Funktionen der angereicherten Population von extrazellulärem Material oder einer Fraktion davon zu bewirken.

11. Pharmazeutische Zusammensetzung nach einem der Ansprüche 1 bis 10, ferner umfassend anderes extrazelluläres Material, eine Zelle, eine membrangebundene Einheit oder eine lipidhaltige Einheit, die mit dem extrazellulären Material oder einer Fraktion davon verschmolzen ist.

12. Pharmazeutische Zusammensetzung nach Anspruch 1 bis 11, wobei das extrazelluläre Material einen durchschnittlichen Partikeldurchmesser oder einen durchschnittlichen hydrodynamischen Durchmesser zwischen 50 und 1000 nm aufweist.

13. Pharmazeutische Zusammensetzung nach einem der Ansprüche 1 bis 12, wobei das extrazelluläre Material eine negative Oberflächenladung oder ein negatives Zetapotential aufweist.

14. Pharmazeutische Zusammensetzung nach einem der Ansprüche 1 bis 13, wobei die pharmazeutische Zusammensetzung in Pflasterform vorliegt.

15. Verfahren zur Anreicherung von Zellen mit extrazellulärem Material, umfassend:
a) Eintauchen der Zellen in eine Lösung mit einer Osmolalität zwischen 380 und 1000 mOsm/kg;
b) Inkubieren der Zellen in der Lösung mit einer Osmolalität zwischen 380 und 1000 mOsm/kg; und
c) Sammeln und Reinigen des von diesen Zellen freigesetzten extrazellulären Materials.

16. Pharmazeutische Zusammensetzung nach einem der Ansprüche 1 bis 14 oder nach dem Verfahren von Anspruch 15, wobei die Zellen mesenchymale Stammzellen umfassen.

## Revendications

1. Composition pharmaceutique comprenant une population enrichie de matériau extracellulaire dérivé de cellules vivantes isolées ou cultivées, dans laquelle ledit matériau extracellulaire est libéré desdites cellules par immersion desdites cellules dans une solution comprenant une osmolalité comprise entre 380 et 1000 mOsm/kg, telle que 480 à 860 mOsm/kg.

2. Composition pharmaceutique selon la revendication 1, dans laquelle ladite solution comprend des composants capables de maintenir la viabilité cellulaire *in vitro,* les composants comprenant des sels de lithium, de sodium, de potassium, de calcium, de magnésium, de chlorure, de phosphate, d'acétate, de bicarbonate, et/ou de citrate.

3. Composition pharmaceutique selon l'une quelconque des revendications 1 et 2, dans laquelle les cellules comprennent des cellules souches adultes et/ou le matériau extracellulaire comprend des vésicules extracellulaires.

4. Composition pharmaceutique selon l'une quelconque des revendications 1 à 3, dans laquelle ladite population enrichie de matériau extracellulaire comprend une molécule thérapeutique.

5. Composition pharmaceutique selon la revendication 4, dans laquelle ladite molécule thérapeutique est CD9, CD63, CD81, le cholestérol, la phosphatidylsérine, ou une combinaison de ceux-ci.

6. Composition pharmaceutique selon la revendication 4, dans laquelle ladite molécule thérapeutique est l'angiopoïétine (Ang-1).

7. Composition pharmaceutique selon la revendication 4, dans laquelle ladite molécule thérapeutique comprend des molécules d'acide nucléique, des polypeptides, des lipides, des hormones, des vitamines, des minéraux, des petites molécules, et des produits pharmaceutiques, ou toute combinaison de ceux-ci.

8. Fraction isolée ou purifiée de la composition pharmaceutique selon l'une quelconque des revendications 1 à 7.

9. Composition pharmaceutique selon l'une quelconque des revendications 1 à 8, dans laquelle ladite population enrichie de matériau extracellulaire ou une fraction de celle-ci est mise en suspension dans un système liquide ou colloïdal, congelée, séchée, lyophilisée, immobilisée sur la surface d'un autre matériau, ou encapsulée dans un autre matériau.

10. Composition pharmaceutique selon l'une quelconque des revendications 1 à 9, dans laquelle la composition pharmaceutique comprend de petites molécules, des agents de contraste, des conservateurs, des stabilisants, des protéines, des lipides, des acides nucléiques, des glucides, leurs dérivés, ou une combinaison de ceux-ci, pour affecter un changement permanent ou temporaire des caractéristiques physico-chimiques, de la biodistribution, de la pharmacocinétique, de la pharmacodynamique, ou des fonctions biologiques de ladite population enrichie de matériau extracellulaire ou d'une fraction de celle-ci.

11. Composition pharmaceutique selon l'une quelconque des revendications 1 à 10, comprenant également un autre matériau extracellulaire, une cellule, un assemblage lié à une membrane, ou un assemblage contenant des lipides, qui est fusionné audit matériau extracellulaire ou à une fraction de celui-ci.

12. Composition pharmaceutique selon les revendications 1 à 11, dans laquelle ledit matériau extracellulaire présente un diamètre moyen de particule ou un diamètre hydrodynamique moyen compris entre 50 et 1000 nm.

13. Composition pharmaceutique selon l'une quelconque des revendications 1 à 12, dans laquelle ledit matériau extracellulaire porte une charge de surface négative, ou un potentiel zêta négatif.

14. Composition pharmaceutique selon l'une quelconque des revendications 1 à 13, dans laquelle la composition pharmaceutique est sous forme de patch.

15. Procédé d'enrichissement de cellules en matériau extracellulaire, comprenant :
a) l'immersion des cellules dans une solution ayant une osmolalité comprise entre 380 et 1000 mOsm/kg ;
b) l'incubation des cellules dans ladite solution ayant une osmolalité comprise entre 380 et 1000 mOsm/kg ; et
c) la collecte et la purification du matériau extracellulaire libéré par lesdites cellules.

16. Composition pharmaceutique selon l'une quelconque des revendications 1 à 14 ou procédé selon la revendication 15, dans lequel les cellules comprennent des cellules souches mésenchymateuses.
